# EUROPEAN PATENT SPECIFICATION

(11) **EP 1 893 761 B1**
(45) Date of publication and mention of the grant of the patent: **05.12.2012**
(21) Application number: 06765647.0
(22) Date of filing: 26.05.2006
(51) Int. Cl.: C12N 15/82, A01H 5/00

(54) **MODULATION OF PLANT GROWTH BY ALTERING AMINO ACID UPTAKE**
MODULATION VON PFLANZENWACHSTUM DURCH VERÄNDERUNG DER AMINOSÄUREAUFNAHME
MODULATION DE LA CROISSANCE DE PLANTE PAR MODIFICATION DU CAPTAGE DES ACIDES AMINES

(30) Priority: 27.05.2005 GB 0510928
(43) Date of publication of application: 05.03.2008
(73) Proprietor: SWETREE TECHNOLOGIES AB, 904 03 Umea (SE)
(72) Inventor: NASHOLM, Torgny Umea Plant Science Centre, S-90183 Umea (SE); SVENNERSTAM, Henrik Umea Plant Science Centre, S-90183 Umea (SE); FORSUM, Oskar Umea Plant Science Centre, S-90183 Umea (SE); GANENTEG, Ulrika Umea Plant Science Centre, S-90183 Umea (SE); ZETHERSTROM, Margareta Umea Plant Science Centre, S-90183 Umea (SE)
(74) Representative: Sutcliffe, Nicholas Robert
(86) International application number: PCT/IB2006/002220
(87) International publication number: WO 2006/126100

(56) References cited:
- WO-A2-94/01559
- WO-A2-03/060133
- WO-A2-03/066879
- WO-A2-03/072792
- US-A- 6 165 792
- US-A1- 2005 044 585
- ERIKSON OSKAR ET AL: "The dsdA gene from Escherichia coli provides a novel selectable marker for plant transformation" PLANT MOLECULAR BIOLOGY, vol. 57, no. 3, February 2005 (2005-02), pages 425-433, XP002407277 ISSN: 0167-4412 cited in the application
- ERIKSON OSKAR ET AL: "A conditional marker gene allowing both positive and negative selection in plants" NATURE BIOTECHNOLOGY, NATURE PUBLISHING GROUP, NEW YORK, NY, US, vol. 22, no. 4, April 2004 (2004-04), pages 455-458, XP002292188 ISSN: 1087-0156 cited in the application
- HIRNER BRIGITTE ET AL: "Developmental control of H+/amino acid permease gene expression during seed development of Arabidopsis" PLANT JOURNAL, vol. 14, no. 5, June 1998 (1998-06), pages 535-544, XP002407278 ISSN: 0960-7412
- KOCH W ET AL: "Reduced amino acid content in transgenic potato tubers due to antisense inhibition of the leaf H+/amino acid symporter StAAP1" PLANT JOURNAL, BLACKWELL SCIENTIFIC PUBLICATIONS, OXFORD, GB, vol. 33, no. 2, January 2003 (2003-01), pages 211-220, XP002266693 ISSN: 0960-7412

## Description

This invention relates to the modulation of the uptake of amino acids from the environment by plants through alterations in the expression of amino acid transporters in order to influence plant growth.

Nitrogen is a mineral nutrient that is needed in large amounts by plants. Sources of nitrogen for plant growth include inorganic nitrogen compounds, such as ammonium and nitrate, and organic nitrogen compounds, including amino acids.

Improving the acquisition of nitrogen by plants is an important objective in increasing crop yields (Britto et al (2004) BioEssays 26:683-692). However, attempts to enhance plant nitrogen acquisition by the increasing expression of nitrogen transporter proteins have been hindered by various factors.

In many plant species, the influx of nitrogen from the environment is inversely related to the nitrogen content of the plant i.e. the greater the nitrogen content of the plant, the lower the uptake of nitrogen. Nitrogen uptake may be down-regulated by the originally transported nitrogen sources, which can accumulate to substantial concentrations within plant cells, or the products of the metabolic assimilation of these ions. This feedback inhibition makes it difficult to increase the plants capacity to abstract nitrogen from the soil.

Furthermore, plant roots often display excess nitrogen-influx capacity and even under nitrogen limitation conditions, a sizable efflux of nitrogen is often observed from root cells back into the external environment. This excess nitrogen-influx capacity indicates that plants take up more nitrogen from the environment than they assimilate or store, and this excess is then expelled back into the environment. The over-expression of nitrogen-influx transporters thus results in increased nitrogen efflux, with no net advantage to the crop.

WO 03/066879 discloses nucleic acid molecules that encode amino acid transporters in a plant, and altering the activity of such amino acid transporters.

WO 94/01559 discloses DNA sequences that contain the coding region of amino acid transporters. The introduction of these sequences in the genome modifies the transfer of metabolites in transgenic plants, plasmids, bacteria, yeasts and plants.

US Patent number 6165792 discloses an isolated nucleic acid fragment encoding an amino acid transporter. US Patent number 6165792 also describes the construction of a chimeric gene encoding all or a portion of the amino acid transporter, in sense or antisense orientation. Altering expression of the chimeric gene results in production of altered levels of the amino acid transporter in a transformed host cell.

WO 03/060133 discloses plants and plant cells which express heterologous D-amino acid metabolising enzymes and may therefore employ D-amino acids as a source of nitrogen.

Amino acids occurring in soil are generally absorbed by plants through the root and, following absorption, metabolised so that the nitrogen carried by the absorbed amino acid is distributed into a range of nitrogen compounds.

The present invention relates to finding that the growth of plants may be directly affected by the altered expression of amino acid transporters that mediate the uptake of amino acids from the environment. This may be useful in altering plant growth characteristics.

As set out in the claims the invention provides a method of increasing the growth of a plant comprising:
increasing the expression of a nucleic acid encoding an amino acid transporter in the plant,
the growth of said plant being increased following said expression.

Expression of a nucleic acid encoding an amino acid transporter may be increased in a plant by recombinant means (i.e. by genetic engineering techniques), for example by expressing a recombinant nucleic acid encoding the amino acid transporter in the plant or by increasing or activating the expression of an endogenous nucleic acid encoding the amino acid transporter in the plant using a recombinant nucleic acid construct, for example by operably linking the nucleic acid with a heterologous promoter (i.e. a promoter with which the nucleic acid is not naturally expressed).

The nucleic acid may be a heterologous nucleic acid. A method of increasing the growth of a plant may, for example, comprise;
expressing a heterologous nucleic acid encoding an amino acid transporter in the plant,
the growth of said plant being increased following said expression.

The plant may be exposed to a growth substrate comprising one or more amino acids, such that the expressed amino acid transporter mediates the uptake of amino acids from the substrate into the plant. Suitable growth substrates are well known in the art and include natural plant growth media, such as soil, peat or compost, or synthetic plant growth media, such as Murashige & Skoog Media, Gamborg's B5 and Chu's N6 (Qbiogene, Irvine CA). The one or more amino acids may be naturally present in the growth medium or may be added to the growth medium. In some embodiments, the one or more amino acids may be the sole nitrogen source in the growth medium.

Suitable amino acids include L- and/or D-enantiomers of amino acids such as His, Glu, Gln, Ala, Lys, Arg, Asp and Ser and the non-chiral amino acid Gly.

In preferred embodiments, amino acids taken up by the plant through the amino acid transporter are metabolised via the nitrogen metabolism pathways of the plant and are not stored or accumulated in seeds or other tissues.

Preferably, the nucleic acid encoding the amino acid transporter is expressed preferentially or specifically in tissues of the plant which contact the growth medium comprising the one or more amino acids. For example, the nucleic acid may be specifically expressed in root tissue of the plant, in particular in root hairs of the plant. Tissue-specific expression of the nucleic acid may be achieved by operably linking the nucleic to one or more tissue-specific regulatory elements, as described below.

The expressed nucleic acid may encode any amino acid transporter which is able to mediate the uptake of an amino acid into the plant from outside the plant (e.g. from the growth medium). For example, the amino acid transporter may mediate the uptake of any amino acid which is naturally present in the growth medium or which is added to the growth medium, L- and/or D-enantiomers of amino acids such as His, Glu, Gln, Ala, Lys, Arg, Asp and Ser and the non-chiral amino acid Gly. The amino acid transporter may be an amino acid transporter which is naturally expressed in the plant (i.e. an endogenous amino acid transporter) or an amino acid transporter which is not naturally expressed in the plant (i.e. a foreign or exogenous amino acid transporter).

Suitable amino acid transporters include plant and non-plant proteins, including bacterial proteins such as E. *coli cycA,* or variants or fragments thereof.

Suitable amino acid transporters may mediate uptake of both D-and L- enantiomers of amino acids (Montamat et al. 1999, Plant Mol Biol 41:259-268).

Lysine/histidine transporters may be used in methods of the invention. Lysine/histidine transporters include the LHT1 amino acid transporters. A suitable LHT1 amino acid transporter may have the sequence of the Arabidopsis thaliana LHT1 amino acid transporter (protein database entry: AAC49885.1 GI: 2576361; nucleic acid database entry (U39782.1 GI:2576360) or may be a variant or fragment thereof. Suitable variants or fragments of an LHT1 amino acid transporter retain the activity of the wild-type sequence to mediate amino acid uptake.

Amino acid permease (AAP) polypeptides may be used in methods of the invention. Amino acid permeases include the AAP1, AAP3, AAP6 and AAP8 amino acid transporters. Suitable AAP polypeptides may have the sequence of the Arabidopsis thaliana AAP1, AAP3, AAP6 or AAP8 transporter (as referenced in Table 2) or may be a variant or fragment thereof. Suitable variants or fragments of an AAP transporter retain the activity of the wild-type sequence to mediate amino acid uptake.

Other suitable amino acid transporters include but are not limited to polypeptides listed in Table 2 or variants or fragments thereof.

A variant of a polypeptide sequence for use in accordance with any aspect of the invention may have one or more of addition, insertion, deletion or substitution of one or more amino acids in the wild-type polypeptide sequence. For example, up to about 5, 10, 15 or 20 amino acids may be altered. Such alterations may be caused by one or more of addition, insertion, deletion or substitution of one or more nucleotides in the encoding nucleic acid. An amino acid sequence variant of a wild-type polypeptide sequence may comprise an amino acid sequence which shares greater than 20% sequence identity with the wild-type sequence, greater than 30%, greater than 35%, greater than 40%, greater than 45%, greater than 55%, greater than 65%, greater than 70%, greater than about 80%, greater than 90% or greater than 95%. The sequence may share greater than 20% similarity with the wild-type sequence, greater than 30% similarity, greater than 40% similarity, greater than 50% similarity, greater than 60% similarity, greater than 70% similarity, greater than 80% similarity or greater than 90% similarity.

Sequence similarity and identity are commonly defined with reference to the algorithm GAP (Wisconsin GCG package, Accelerys Inc, San Diego USA). GAP uses the Needleman and Wunsch algorithm to align two complete sequences that maximizes the number of matches and minimizes the number of gaps. Generally, default parameters are used, with a gap creation penalty = 12 and gap extension penalty = 4. Use of GAP may be preferred but other algorithms may be used, e.g. BLAST (which uses the method of Altschul et al. (1990) J. Mol. Biol. 215: 405-410), FASTA (which uses the method of Pearson and Lipman (1988) PNAS USA 85: 2444-2448), or the Smith-Waterman algorithm (Smith and Waterman (1981) J. Mol Biol. 147: 195-197), or the TBLASTN program, of Altschul et al. (1990) supra, generally employing default parameters. In particular, the psi-Blast algorithm (Nucl. Acids Res. (1997) 25 3389-3402) may be used. Sequence identity and similarity may also be determined using Genomequest™ software (Gene-IT, Worcester MA USA).

Sequence comparisons are preferably made over the full-length of the relevant sequence described herein.

Similarity allows for "conservative variation", i.e. substitution of one hydrophobic residue such as isoleucine, valine, leucine or methionine for another, or the substitution of one polar residue for another, such as arginine for lysine, glutamic for aspartic acid, or glutamine for asparagine.

The term "heterologous" indicates an element which is not naturally occurring in a particular context. For example, a nucleotide or polypeptide sequence which is heterologous to a plant-cell may be non-naturally occurring in cells of that type, variety or species. Similarly, a heterologous regulatory element or promoter may not be naturally associated with a nucleic acid coding sequence in nature. Heterologous elements may be combined or associated by genetic engineering or recombinant means. In some embodiments, a heterologous nucleic acid or regulatory element may be a recombinant nucleic acid or regulatory element.

Nucleic acid encoding an amino acid transporter may be contained on a nucleic acid construct or vector. The construct or vector is preferably suitable for transformation into and/or expression within a plant cell. In some embodiments, the construct or vector comprising the nucleic acid does not include a promoter or other regulatory sequence, for example if the vector is to be used to introduce the nucleic acid into cells for recombination into the genome. In other embodiments, the nucleic acid encoding the amino acid transporter is operatively linked to a regulatory element. The regulatory element is preferably heterologous or foreign to the nucleic acid encoding the amino acid transporter.

Preferably, the regulatory element is plant specific. A plant specific regulatory sequence or element is a sequence which preferentially directs the expression of a nucleic acid within a plant cell relative to other cell types. For example, expression from such a sequence may be reduced or abolished in non-plant cells, such as bacterial or mammalian cells. Such regulatory sequences may provide for efficient expression within a plant cell. Many suitable regulatory sequences are known in the art and may be used in accordance with the invention. Examples of suitable regulatory sequences may be derived from a plant virus, for example the Cauliflower Mosaic Virus 35S (CaMV 35S) gene promoter that is expressed at a high level in virtually all plant tissues (Benfey et al, (1990) EMBO J 9: 1677-1684). Leaf specific promoters may also be used (see for example Lagrange et al Plant Cell. 1997 9 (8): 1469-1479). Other suitable constitutive regulatory elements include the cauliflower mosaic virus 19S promoter; the Figwort mosaic virus promoter; and the nopaline synthase (nos) gene promoter (Singer et al., Plant Mol. Biol. 14:433 (1990); An, Plant Physiol. 81:86 (1986)).

In some preferred embodiments, a tissue specific regulatory element may be employed, in particular a root-specific regulatory element, for example an element which specifically drives expression of the amino acid transporter in root hair.

Suitable root-specific regulatory elements include the LeExtl promoter (Bucher et al Plant Physiology, March 2002, Vol. 128, pp. 911-923) and the ROP2 promoter (Xu et al The Plant Cell, Vol. 17, 525-536).

In some embodiments, a regulatory sequence operatively linked to the nucleic acid sequence may be inducible. Inducible promoters are well known in the art and include, for example the HSP promoter (Severin K. and Schöffl F. 1990. Plant Mol. Biol. 15: 827-833). In essence, expression under the control of an inducible promoter is "switched on" or increased in response to an applied stimulus (which may be generated within a cell or provided exogenously). The nature of the stimulus varies between promoters. Whatever the level of expression is in the absence of the stimulus, expression from any inducible promoter is increased in the presence of the correct stimulus. The preferable situation is where the level of expression of the amino acid transporter is increased in the presence of the relevant stimulus by an amount which is effective to alter amino acid uptake and thereby modulate growth.

Constructs and vectors may further comprise selectable genetic markers consisting of genes that confer selectable phenotypes such as resistance to antibiotics such as kanamycin, hygromycin, phosphinotricin, chlorsulfuron, methotrexate, gentamycin, spectinomycin, imidazolinones, glyphosate and D-amino acids.

Those skilled in the art can construct vectors and design protocols for recombinant gene expression, for example in a microbial or plant cell. Suitable vectors can be chosen or constructed, containing appropriate regulatory sequences, including promoter sequences, terminator fragments, polyadenylation sequences, enhancer sequences, marker genes and other sequences as appropriate. For further details see, for example, Molecular Cloning: a Laboratory Manual: 3rd edition, Sambrook et al, 2001, Cold Spring Harbor Laboratory Press and Protocols in Molecular Biology, Second Edition, Ausubel et al. eds. John Wiley & Sons, 1992. Specific procedures and vectors previously used with wide success upon plants are described by Bevan, Nucl. Acids Res. (1984) 12, 8711-8721), and Guerineau and Mullineaux, (1993) Plant transformation and expression vectors. In: Plant Molecular Biology Labfax (Croy RRD ed) Oxford, BIOS Scientific Publishers, pp 121-148.

When introducing a chosen gene construct into a cell, certain considerations must be taken into account, well known to those skilled in the art. The nucleic acid to be inserted should be assembled within a construct that contains effective regulatory elements that will drive transcription. There must be available a method of transporting the construct into the cell. Once the construct is within the cell membrane, integration into the endogenous chromosomal material either will or will not occur. Finally, the target cell type is preferably such that cells can be regenerated into whole plants.

Techniques well known to those skilled in the art may be used to introduce nucleic acid constructs and vectors into plant cells to produce transgenic plants with the properties described herein.

A plant comprising a nucleic acid which encodes an amino acid transporter, for example a heterologous nucleic acid, may be produced by transforming a plant cell with the nucleic acid and regenerating the plant from the cell.

Agrobacterium transformation is one method widely used by those skilled in the art to transform plant species. Production of stable, fertile transgenic plants is now routine in the art:(Toriyama, et al. (1988) Bio/Technology 6; 1072-1074; Zhang, et al. (1988) Plant Cell Rep. 7, 379-384; Zhang, et al. (1988) Theor Appl Genet 76, 835-840; Shimamoto, et al. (1989) Nature 338, 274-276; Datta, et al. (1990) Bio/Technology 8, 736-740; Christou, et al. (1991) Bio/Technology 9, 957-962; Peng, et al. (1991) International Rice Research Institute, Manila, Philippines 563-574; Cao, et al. (1992) Plant Cell Rep. 11, 585-591; Li, et al. (1993) Plant Cell Rep. 12, 250-255; Rathore, et al. (1993) Plant Molecular Biology 21, 871-884; Fromm, et al. (1990) Bio/Technology 8, 833-839; Gordon-Kamm, et al. (1990) Plant Cell 2, 603-618; D'Halluin, et al. (1992) Plant Cell 4, 1495-1505; Walters, et al. (1992) Plant Molecular Biology 18, 189-200; Koziel, et al. (1993) Biotechnology 11, 194-200; Vasil, I. K. (1994) Plant Molecular Biology 25, 925-937; Weeks, et al. (1993) Plant Physiology 102, 1077-1084; Somers, et al. (1992) Bio/Technology 10, 1589-1594; WO92/14828; Nilsson, O. et al (1992) Transgenic Research 1, 209-220).

Other methods, such as microprojectile or particle bombardment (US 5100792, EP-A-444882, EP-A-434616), electroporation (EP 290395, WO 8706614), microinjection (WO 92/09696, WO 94/00583, EP 331083, EP 175966, Green et al. (1987) Plant Tissue and Cell Culture, Academic Press), direct DNA uptake (DE 4005152, WO 9012096, US 4684611), liposome mediated DNA uptake (e.g. Freeman et al. Plant Cell Physiol. 29: 1353 (1984)), or the vortexing method (e.g. Kindle, PNAS U.S.A. 87: 1228 (1990d)) may be preferred where Agrobacterium transformation is inefficient or ineffective, for example in some gymnosperm species.

Physical methods for the transformation of plant cells are reviewed in Oard, 1991, Biotech. Adv. 9:1-11.

Alternatively, a combination of different techniques may be employed to enhance the efficiency of the transformation process, e.g. bombardment with Agrobacterium coated microparticles (EP-A-486234) or microprojectile bombardment to induce wounding followed by co-cultivation with Agrobacterium (EP-A-486233).

Following transformation, a plant may be regenerated, e.g. from single cells, callus tissue or leaf discs, as is standard in the art. Almost any plant can be entirely regenerated from cells, tissues and organs of the plant. Available techniques are reviewed in Vasil et al., Cell Culture and Somatic Cell Genetics of Plants, Vol I, II and III, Laboratory Procedures and Their Applications, Academic Press, 1984, and Weissbach and Weissbach, Methods for Plant Molecular Biology, Academic Press, 1989.

The particular choice of a transformation technology will be determined by its efficiency to transform certain plant species as well as the experience and preference of the person practising the invention with a particular methodology of choice. It will be apparent to the skilled person that the particular choice of a transformation system to introduce nucleic acid into plant cells is not essential to or a limitation of the invention, nor is the choice of technique for plant regeneration.

Disclosed herein is a method of selectively growing a plant comprising;
providing a plant with reduced levels or activity of an amino acid transporter which mediates uptake of a D-amino acid and treating the plant with the D-amino acid,
said treatment providing selective growth of the plant.

Suitable D-amino acids are toxic to the plant and include a D-amino acid selected from the group consisting of D-arg, D-glu, D-ala, D-asp, D-cys, D-gln, D-his, D-ile, D-leu, D-lys, D-met, D-asn, D-phe, D-pro, D-ser, D-thr, D-trp, D-tyr or D-val. For example, the D-amino acid may be D-ala, D-ser, D-arg, D-glu, in particular D-ala or D-Ser.

The level or activity of an amino acid transporter may be reduced by any convenient method. For example, levels may be reduced by expression of a suppressor nucleic acid, for example an anti-sense, sense or RNAi nucleic acid, by mutation of a nucleic acid encoding the amino acid transporter, in cells of the plant or an ancestor thereof or by Virus Induced Gene Silencing (VIGS).

The level or activity of an amino acid transporter mediating uptake of amino acids from the external environment may be reduced in the roots of the plant, preferably specifically in the roots of the plant and not in other tissues. The plant may then be treated with a D-amino acid by growing the plant in a medium comprising the D-amino acid.

The level or activity of an amino acid transporter mediating uptake of amino acids from the external environment may be reduced in the above ground portion of the plant, preferably specifically in the above ground portion of the plant and not in the roots of the plant or in other tissues. The plant may then be treated with a D-amino acid by contacting the above ground portion of the plant with a composition comprising the D-amino acid.

Suitable amino acid transporters whose level or activity may be reduced in accordance with the present methods are described in more detail above.

A plant having a reduced level or activity of an amino acid transporter mediating uptake of amino acids from the external environment may be produced by mutating the nucleic acid sequence encoding an amino acid transporter, or a regulatory element thereof, in a plant cell or plant tissue such as a seed, and regenerating a plant from the cell or tissue. The nucleic acid may be mutated by any convenient method, including targeting mutation methods, such as T-DNA insertion and methods promoting random sequence alterations in the genome such as EMS treatment. Suitable methods are well known in the art. A plant having a reduced level or activity of an amino acid transporter mediating uptake of amino acids from the external environment may be produced by expressing a suppression nucleic acid construct such as a sense, anti-sense or RNAi nucleic acid comprising or consisting of all or part of the nucleic acid sequence encoding the transporter. Methods for suppressing the expression of nucleic acids using sense, anti-sense or RNAi techniques are well known in the art.

A method may comprise reducing the level or activity of the amino acid transporter which mediates uptake of amino acids from the external environment in the plant by expressing a suppressor nucleic acid construct.

Disclosed herein is a method of increasing the sensitivity of a plant to a toxic D-amino acid comprising;
expressing in the plant a heterologous nucleic acid encoding an amino acid transporter which mediates uptake of the toxic D-amino acid,
said expression increasing the sensitivity of the plant to toxic D-amino acid.

A method of selectively inhibiting the growth of a plant may comprise;
expressing in the plant a heterologous nucleic acid encoding an amino acid transporter which mediates uptake of a toxic D-amino acid, and;
treating said plant with a composition comprising the D-amino acid,
said expression increasing the uptake of the toxic D-amino acid.

Suitable toxic D-amino acids include a D-amino acid selected from the group consisting of D-arg, D-glu, D-ala, D-asp, D-cys, D-gln, D-his, D-ile, D-leu, D-lys, D-met, D-asn, D-phe, D-pro, D-ser, D-thr, D-trp, D-tyr or D-val. For example, the D-amino acid may be D-ala, D-ser, D-arg or D-glu, in particular D-ala or D-ser (Erikson et al. 2004, Nature Biotech 22: 455-458).

Methods and means for expressing amino acid transporters in plants are described above.

The amino acid transporter that mediates uptake of amino acids from the external environment may be expressed in the roots of the plant, preferably specifically in the roots of the plant and not in other tissues. The plant may then be treated with a D-amino acid by growing the plant in a medium comprising the D-amino acid.

The amino acid transporter that mediates uptake of amino acids from the external environment may be expressed in the above ground portion of the plant, preferably specifically in the above ground portion of the plant and not in the roots of the plant or in other tissues. The plant may then be treated with a D-amino acid by contacting the above ground portion of the plant with a composition comprising the D-amino acid.

Suitable amino acid transporters which may be expressed in accordance with the present methods are described in more detail above.

Disclosed herein are plants which are able to metabolise one or more D-amino acids. Suitable plants may, for example, be engineered to express a heterologous polypeptide which has an enzymatic activity which converts the one or more D-amino acids into a plant-metabolisable substrate.

A method of selectively growing a plant that metabolises a D-amino acid may comprise;
expressing in the plant a heterologous nucleic acid encoding an amino acid transporter which mediates uptake of the D-amino acid, and;
treating said plant with a composition comprising the D-amino acid,
said expression providing selective growth of said plant.

The D-amino acid may be D-arg, D-glu, D-ala, D-asp, D-cys, D-gln, D-his, D-ile, D-leu, D-lys, D-met, D-asn, D-phe, D-pro, D-ser, D-thr, D-trp, D-tyr or D-val; preferably, D-ser, D-asn, D-ala, D-ile, D-glu, D-arg, D-lys, D-his or D-asp; more preferably D-ala, D-ile or D-ser.

Other D-amino acids may include non-protein dextrotatory amino acids, precursors of dextrotatory amino acids, such as D-ornithine and D-citrulline, and dextrotatory amino acid derivatives. Such may be used as a plant nitrogen source only after conversion into a suitable metabolisable form by a D-amino acid metabolising enzyme.

The amino acid transporter may be specifically expressed in the roots of the plant. The plant may then be treated with a D-amino acid by growing the plant in a medium comprising the D-amino acid.

The amino acid transporter may be specifically expressed in the above ground portion of the plant. The plant may then be treated with a D-amino acid by contacting the above-ground portion of the plant with a composition comprising the D-amino acid, for example by spraying.

A plant that metabolises a D-amino acid may be produced in accordance with known methods by transforming a plant cell with a nucleic acid encoding a polypeptide with D-amino acid metabolising activity and regenerating the plant from the cell.

A polypeptide with D-amino acid metabolising activity is a polypeptide that converts a D-amino acid substrate into products which are substrates for endogenous plant enzymes (i.e. they can be metabolised by plants), in particular non-dextrotatory compounds i.e. non-chiral or L-compounds. The D-amino acid metabolising polypeptide therefore catalyses the biochemical conversion of the D-amino acid substrate into products, for example non-dextrotatory products, which may be used by plants as sources of nutrients, in particular as sources of nitrogen.

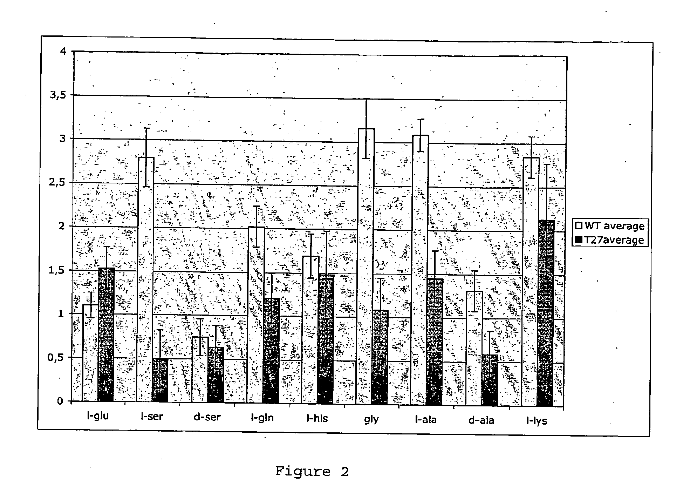

Suitable D-amino acid metabolising polypeptides include, for example, oxidases, racemases, decarboxylases, transaminases or dehydratases (also called ammonia lyases). Oxidases catalyse the conversion of a D-amino acid into NH₄⁺, a keto acid (depending on the D-amino acid substrate) and H₂O₂ (see figure 2). Racemases convert a D-amino acid into the corresponding L-amino acid form. L-amino acids are useful as a nitrogen source for plants. Decarboxylases convert a D-amino acid into a Y-amino acid which can be metabolised by plants. Transaminases convert a D-amino acid into a different L or D amino acid form. L-amino acids can then be metabolised directly whilst D-amino acids can undergo further conversion into metabolisable forms. Dehydratases catalyse the conversion of a hydroxy D-amino acid such as D-ser or D-thr into NH₄⁺, a keto acid (depending on the D-amino acid substrate) and H₂O. Dehydrogenases catalyse the conversion of a D-amino acid into NH₄⁺, a keto acid (depending on the D-amino acid substrate) and H₂O_{.}

A suitable D-amino acid metabolising polypeptide may be a eukaryotic enzyme, for example from a yeast (e.g. *Rhodotorula* gracilis), fungus, or animal or it may be a prokaryotic enzyme, for example, from a bacterium such as *Escherichia* coli. Examples of suitable polypeptides which metabolise D-amino acids are shown in Table 1.

The D-amino acid metabolising polypeptide is a dehydratase, for example D-ser ammonia lyase (formerly known as D-ser dehydratase) or an oxidase, for example D-amino acid oxidase.

Other suitable D-amino acid metabolising polypeptides may be variants or fragments of polypeptides exemplified in Table 1. Variants or fragments of wild-type polypeptide sequences are described elsewhere herein.

A skilled person is readily able to determine using routine techniques whether a polypeptide possesses a D-amino acid metabolising activity (i.e. is a D-amino acid metabolising enzyme), as described above.

The identity of the D-amino acid in the medium is determined by the specificity of the heterologous D-amino metabolising polypeptide which is expressed by the plant or plant cell i.e. the medium contains the D-amino acid substrate of the heterologous D-amino metabolising polypeptide. For example, where heterologous D-serine dehydratase is expressed by a plant, D-serine is present in the growth medium.

Expression of the amino acid transporter in a plant which metabolises D-amino acids may display increased growth on media comprising D-amino acids relative to similar plants not expressing the amino acid transporter, in particular where the D-amino acid represents the sole nitrogen source.

The amino acid transporter mediates the uptake of the D-amino acid from the medium into the plant to allow reaction with the D-amino metabolising polypeptide. Thus, the identity of the amino acid transporter expressed by the plant is determined by the D-amino acid in the medium, which is, in turn, determined by the specificity of the D-amino metabolising polypeptide.

Suitable amino acid transporters are described in more detail above and include plant and non-plant amino acid transporters, for example, bacterial amino acid transporters such as the E.coli cycA amino acid transporter, which mediates transport of Glycine, D-Alanine and D-Serine.

The accumulation of products of D-amino acid metabolism, including for example various keto-acids and the production of H₂O₂ by D-amino acid oxidases, may be deleterious to transgenic plants. Methods described herein may therefore be useful in selectively removing transgenic plants from mixed populations or removing hybrids between transgenic plants and wild type plants from natural populations.

A method of inhibiting the growth of a plant that metabolises a D-amino acid may comprise;
expressing in the plant an amino acid transporter which mediates the uptake of the D-amino acid, and;
treating the plant with the D-amino acid, said treatment inhibiting the growth of the plant.

A suitable D-amino acid may be non-toxic to the plant, but may be metabolised by the plant into a toxic product. D-amino acids which exhibit low toxicity to wild type plants, for example D-ile, D-val, D-asn, or D-gln, but which, following metabolism in transgenic plants containing exogenous D-amino acid oxidases, lead to the production of toxic keto acids and/or toxic H₂O₂, are particularly suitable for use in such methods.

A plant that metabolises a D-amino acid may be produced by transforming a plant cell with a nucleic acid encoding a polypeptide that metabolises a D-amino acid and regenerating the plant from the cell.

An amino acid transporter may be expressed using any convenient technique, as described above. A suitable amino acid transporter mediates uptake of the D-amino acid from the medium. Suitable amino acid transporters are described in more detail above and include plant and non-plant transporters, such as the E. coli cycA polypeptide.

The plant may oxidise a D-amino acid, for example by expression of a D-amino acid oxidase. D-amino acids such as D-ile and D-val have low toxicity to wild type plants, but are metabolised into the toxic keto acids 3-methyl-2-oxopentanoate and 3-methyl-2-oxobutanoate, respectively by plants expressing a D-amino acid oxidase. The exposure of plants expressing a D-amino acid oxidase to compounds such as D-ile or D-val, derivatives of these or other compounds that upon activity of the D-amino acid oxidase produces toxic products can thus be used to selectively inhibit growth of such plants.

The addition of a D-amino acid may therefore be used to inhibit or reduce the growth or viability of a transgenic plant which has a D-amino acid oxidase activity.

Disclosed herein are methods of producing transgenic plants which are useful in the present methods. A plant suitable for use in the methods described herein may be produced by;
providing a plant cell which comprises a heterologous nucleic acid which encodes a polypeptide which metabolises a D-amino acid,
transforming the plant cell with a nucleic acid which encodes an amino acid transporter which mediates the uptake of the D-amino acid; and,
regenerating said plant from said cell.

The plant may be regenerated on a substrate which comprises a D-amino acid, which may, for example, be the sole source of nitrogen in the medium.

Also disclosed herein is a plant cell comprising a first heterologous nucleic acid which encodes a polypeptide which metabolises a D-amino acid,
wherein the plant cell further comprises a second heterologous nucleic acid which encodes an amino acid transporter which mediates the uptake of the D-amino acid.

The first and second nucleic acids may be contained in nucleic acid constructs or vectors. The first and the second nucleic acid may be contained in the same expression vector or different expression vectors in said cell.

The first and the second nucleic acids may be extra-chromosomal or may be integrated into the chromosome of the cell.

There may be more than one heterologous nucleotide sequence per haploid genome. This, for example, enables increased expression of the gene product compared with endogenous levels.

The nucleic acids may be operatively linked to regulatory elements for expression. Suitable elements are described above and include externally inducible gene promoters, for example to place expression under the control of the user.

A plant cell may contain the first and second nucleic acid sequences as a result of the introduction of the nucleic acid sequences into the cell or an ancestor cell.

As described herein, expression of the amino acid transporter and D-amino acid metabolising polypeptide from the encoding nucleic acid may be used to select for transformants that contain the first and second nucleic acids as described herein. A vector may additionally comprise a selectable genetic marker consisting of a chimaeric gene that confers a selectable phenotype such as resistance to an antibiotic such as kanamycin, hygromycin, phosphinotricin, chlorsulfuron, methotrexate, gentamycin, spectinomycin, imidazolinones and glyphosate. This allows for a second level of selection, if desired, in addition to selection using the amino acid transporter and D-amino acid metabolising polypeptide.

A plant cell as described herein may be comprised in a plant, a plant part or a plant propagule, or an extract or derivative of a plant as described below. A plant may be regenerated from one or more cells as described above. Off-spring or descendants of the regenerated plant may then be sexually or asexually propagated or grown.

A plant comprising a cell as described herein may express the encoded D-amino acid metabolising polypeptide and amino acid transporter and so may have enhanced D-amino acid tolerance and may be selectable using D-amino acid treatment.

Plants which include a plant cell as described herein are also provided, along with any part or propagule thereof, seed, selfed or hybrid progeny and descendants. Particularly provided are transgenic monocotyledons, dicotelydons, gymnosperms, angiosperms and algae, ferns and mosses. Of particular interest are transgenic higher plants, especially agricultural and forest crops, for example cereals, trees and ornamental flowers, which have been engineered to carry a nucleic acid construct as described above.

Examples of suitable plants include tobacco, cucurbits, carrot, vegetable brassica, melons, capsicums, grape vines, lettuce, strawberry, crop plants such as oilseed brassica, sugar beet, wheat, barley, maize and rice, soyabeans, peas, sorghum, sunflower, tomato, potato, pepper, chrysanthemum, carnation, poplar, eucalyptus, cotton, rubber, linseed, bamboo, acacia, fruit bearing plants such as apple, plum, pear, banana, orange, kiwi, lemon, cherry, grapevine and fig, hemp, spruce, birch, peanuts, rye and pine.

In addition to a plant, disclosed herein is any clone of such a plant, seed, selfed or hybrid progeny and descendants, and any part or propagule of any of these, such as cuttings and seed, which may be used in reproduction or propagation, sexual or asexual. Also disclosed herein is a plant which is a sexually or asexually propagated off-spring, clone or descendant of such a plant, or any part or propagule of said plant, off-spring, clone or descendant.

Control experiments may be performed as appropriate in the methods described herein. The performance of suitable controls is well within the competence and ability of a skilled person in the field.

Various further aspects and embodiments of the present invention will be apparent to those skilled in the art in view of the present disclosure.

Certain aspects and embodiments of the invention will now be illustrated by way of example and with reference to the figures described above and tables described below.

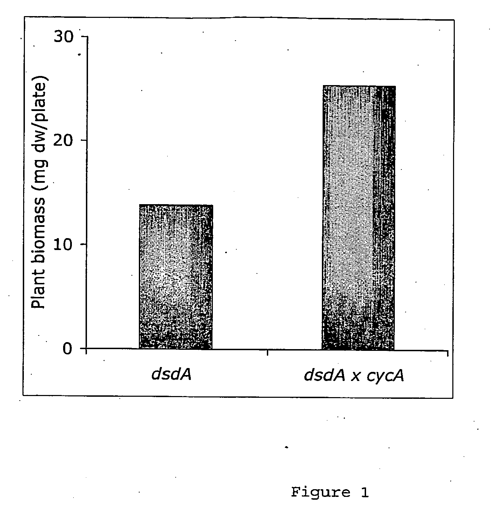

Figure 1 shows growth of A. *thaliana* plants on 1/2 strength MS media in which all nitrogen compounds had been exchanged for 3mM D-serine. Growth of plants carrying the dsdA gene encoding D-serine ammonia lyase from E.coli was compared with that of plants carrying both dsdA and the cycA gene from E.coli and encoding the D-serine/D-alanine transporter.
Figure 2 shows uptake rates of amino acids by A. *thaliana* measured as rates of depletion of an external solution. Wild type plants of ecotype Col-0 are compared with plants carrying a T-DNA insertion in the LHT1 gene, encoding the Lysine-Histidine nol amino acid transporter.

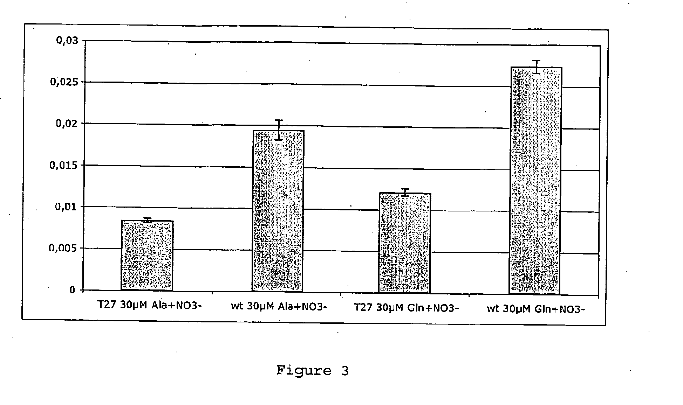

Figure 3 shows the contribution of L-alanine N and L-glutamine N (Ala and Gln respectively) to the N nutrition of sterile grown wild type Arabidopsis thaliana plants (wt) and plants lacking the expression of the lysine-histidine transporter 1 (T27). The X-axis is the fraction of N originating from amino acids.

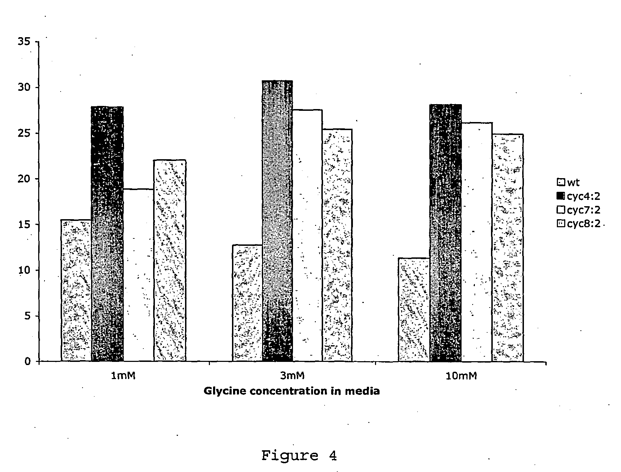

Figure 4 shows the growth of wild type (wt) and transgenic A. thaliana expressing the bacterial cycA transporter on different concentrations of Glycine. Plants were grown in sterile agar media based on ½ strength MS media in which nitrogen was supplied as Glycine only.Cyc4:2, cyc7:2 and cyc8:2 refers to three different transgenic lines.

### Experiments

### Materials and Methods

### Arabidopsis transformation with cycA

DNA manipulations were performed by standard techniques (Sambrook and Russell, 2001) The *E.* coli gene cycA was cloned by PCR using PCR primers 5-
GGGGACAAGTTTGTACAAAAAAGCAGGCTCATGGTAGATCAGGTAAAAGTCGTT and 5'-GGGGACCACTTTGTACAAGAAAGCTGGGTGGTTATTTCCGCAGTTCAGC, the PCR fragments were recombinated into the pDONR and subsequently recombinated into the CaMV 35S expression cassette of the binary vector pH7WG2D. The vector was analysed by sequencing and enzymatic restriction.

Transgenic *A. thaliana plants* carrying the *dsdA* gene from *E.coli* (Erikson et al. 2005, Plant Mol Biol 57, 425-433) were transformed with *Agrobacterium tumefaciens* strain GV3101::pMP90 RK through *in planta* vacuum infiltration as described by Clough and Bent (1998). T₁ seeds were selected on hygromycin. All individual lines isolated were screened with PCR, amplifying the *cycA* gene to confirm integration of the transgene.

### Growth of plants on Petri dishes

Nitrogen free, half strength MS with 0.5% w/v agar and 0.5% w/v sucrose and buffered to pH 5.8 with MES-buffer (2N-morpholinoethanesulfonic acid) was used in all screens and experiments. This media was amended with different N sources as described below.

### Selection of EMS and T-DNA mutants

Except for wild type Arabidopsis thaliana (Col-0), two types of Arabidopsis were used in the experiments; T-DNA insertional mutants and ethyl methane sulfonate (EMS) mutants.

Seeds of 77 T-DNA mutant lines, representing mutations in 46 individual genes encoding or putatively encoding amino acid transporters were obtained from Nottingham seed stock center (NASC). Approximately 1 gram of EMS seeds were obtained from Lehle seed.

Screening for mutations in root absorption of amino acids was performed through spreading seeds on plates containing toxic levels of D-amino acids.

Plates for screening of mutant lines contained the standard media described above amended either with 3 mM D-alanine or 3 mM D-serine. D-amino acids were filter-sterilised and added after autoclaving. Seeds were surface-sterilized and sown on Petri plates that were sealed with gas-permeable tape. All seeds were cold-treated for two days at 4°C after sowing to uniform germination.

A number of EMS plants and one T-DNA insertion line were identified as D-alanine resistant while none of the lines were found to be resistant to D-serine. The EMS plants were moved to soil and were self propagated. Only one EMS line was found to be resistant to D-alanine.

The knockout line surviving D-alanine corresponded to an insertion of T-DNA in the exon of the Lysine Histidine Transporter 1 (LHT1). After the first selection, the LHT1 mutant was subjected to genetical analysis. PCR was used to check whether the insertional mutation was recessive or dominant. PCR analysis showed insertion homozygosity in the plants that survived D-alanine selection, furthermore, the offspring of heterozygous parent plants segregated 3 sensitive to 1 resistant on D-alanine, indicating that the mutation was recessive.

To investigate whether the EMS-mutant and the T-DNA insertional mutants were allelic, the two different mutant lines were crossed. The progeny (T1) of this cross was found to be resistant to D-alanine. Surviving plants were also subjected to PCR analysis that showed that the T-DNA insertions were heterozygous, corroborating that the mutants in fact were allelic.

Sequencing of the LHT1 gene of the EMS mutant identified as D-alanine resistant showed a single g/a nucleotide substitution, which based on TAIR genemodel At5g40780.1 resulted in a change of the native trp66 codon to a stop codon, thereby abolishing the production of LHT1 also in this plant.

### ¹⁵N experiments with LHT1 mutant lines

Wild type seeds and seeds from T-DNA and EMS-lines were sown on agar plates containing ½ strength MS medium with N supplied as a mixture of 3 mM NO₃⁻ and either 30*µ*M L-¹⁵N glutamine, 30*µ*M L-¹⁵N alanine or 30*µ*M L-¹⁵N lysine. Each plate contained 100ml medium. The ¹⁵N concentration of the supplied amino acids were >98% for L-alanine and > 49% for L-glutamine and L-lysine. Eight replicate plates with three seeds each were prepared for each plant type and treatment. Agar plates were incubated in a cold room for two days and then transferred to a climate chamber; 16h light period temp 24 dgr C.

After 14 days, five randomly selected plates were harvested. Roots were rinsed and cleaned thoroughly three times in 0.5 mM CaCl₂ to remove ¹⁵N from the surfaces. Plants were dried at 60°C overnight, weighed and homogenized. Finally, samples were analyzed for total N content and ¹⁵N abundance on a Europa Scientific Isotope Ratio Mass Spectrometer.

### Growth experiments

### Growth of dsdA and cycAxdsdA plants

Plants were grown on ½ strength N free MS media described above and amended with 3 mM D-serine as the sole N source. Plants were grown for 20 days in a growth room under the standard conditions described above.

### Growth of LHT1 mutant lines

Wild type seeds and seeds from T-DNA and EMS-lines were sown on N-free ½ strength MS media described above and amended with either 3mM NO₃⁻; 1.5mM L-glutamine + 3mM NO₃⁻ or 3mM L-alanine + 3mM NO₃⁻. Eight replicate plates, each containing 25ml medium with three seeds each was prepared for each seed line and treatment. After sowing, the agar plates were incubated in a cold room for two days and then transferred to a climate chamber, 16h light period and 24 dgr C. After 20 days, plants were harvested, dried at 60°C and weighed.

### Uptake experiment with LHT1 mutant lines

Wild type seeds and seeds from T-DNA and EMS-lines were sown in agar-filled 1.5 ml Eppendorf tubes. The cap and bottom of each vial were cut off, and the vial placed in Eppendorf vial holders put into growth boxes with nutrient solution. The purpose with this setup was to enable contact between the agar in the tubes and the solution in the box, so that roots were able to grow into the solution. The growth boxes were filled with a nutrient solution containing: 1mM L-Gly, 1mM NH₄NO₃, 0.6mM K₂HPO₄, 0.3mM CaSO₄, 0.55mM MgSO₄, 35*µ*M KCl, 50*µ*M FeNa-EDTA, 35*µ*M H₃BO₃, 7*µ*M MnSO₄, 1.5*µ*M ZnSO₄, 1*µ*M CuSO₄, 0.05*µ*M (NH₄)₆Mo₇O₂₄ and buffered to pH 5.8 with MES.

After sowing, the boxes were incubated in a cold room for two days and then transferred to a climate chamber; 8h light period temp 24/18 dgr (day/night).

After 18 days the healthiest plants were selected and the identified plants transferred to individual boxes. During the first 18 days, aeration of the boxes was accomplished through diffusion via a filter in the lid but for the remainder (after 18 days) of the hydroculture the boxes were aerated by pumping sterile-filtered air trough a syringe that was injected through the lid and in contact with the nutrient solution. Throughout the experiment, growth units that showed signs of infection were discarded. After 39 days of growth, the largest plants were selected for the uptake experiment. Three plants of each line were used in the uptake experiments.

The uptake experiment was carried out in custom made plates, each with a drilled hole that fitted a hollow plastic cylinder that was designed to fixate an Eppendorf vial. This setup enabled an experiment in which evaporation was minimized. The plastic cylinder with Eppendorf tubes (containing a plant) was fitted to the plate so that the root, but not the shoot was in contact with the uptake solution. Moreover, care was taken to ensure that the Eppendorf tube was no in contact with the solution. The uptake solution was identical to the nutrient solution used during plant cultivation but all nitrogen sources were replaced with a mixture of amino acids composed of 25*µ*M of each of the following amino acids D-ala, L-ala, D-ser, L-ser, L-lys, L-his, L-gln, L-glu and gly. Each plate was filled with 10ml of this solution.

The uptake experiment had a duration of 5h with sampling every hour and was carried out in a climate chamber on a shaking table. At each sampling, 120*µ*l solution was withdrawn, transferred to a Eppendorf vial, and frozen immediately.

After 5h the remaining solution was weighed to determine the evapotranspirational loss of water. Plant roots were dried and weighed. Uptake rates (µmol/g DW root/h) were calculated as the average of the concentration differences between each sampling (with original concentration as the first reference). In the calculations evapotranspirational loss was compensated for, assuming that evapotranspiration was linear over time.

### Analyses

D- and L- amino acids were analysed by RP-HPLC of their ortho-phtaldialdehyde derivatives according to Brückner et al. 1991 (Chromatographia, 32: 383-388).

### Results

### Altered growth of plants through altered expression of amino acid transporters

Expression of D-amino acid metabolising enzymes in plants abolishes the negative effect of D-amino acids and enables for plants to use these compounds as nitrogen sources for growth (Erikson, O et al 2004 Nature Biotechnology, 22: 455-458.

Plants were grown on D-serine as the sole source of nitrogen. D-serine mediated growth of dsdA plants carrying the E.coli gene cycA, encoding the D-serine/D-alanine transporter was compared with plants only carrying the dsdA gene (Fig 1). It was shown that D-serine mediated growth was drastically increased through expression of the cycA gene. This shows that plant growth on amino acids can be increased by expression of a heterologus gene encoding an amino acid transporter.

In a second experiment, the importance of expression of amino acid transporters for amino acid mediated growth was shown. Firstly, functional characterization of LHT1 knockout mutants regarding the rate of root uptake of L-amino acids showed that a number of L-amino acids were absorbed at lower rates compared to wild-type plants (Fig 2).

Secondly, growth of plants defective in the LHT1 gene either caused by a T-DNA insertion (line T27) or caused by random mutagenesis following EMS treatment and selection on D-alanine (line EMS-A) was significantly lower on mixtures of nitrate and amino acids compared to that of wild-type plants. Growth of LHT1 defective plants on nitrate as the sole nitrogen source was, however, not different from wild-type plants.

The contribution of L-alanine N and L-glutamine N (Ala and Gln respectively) to N nutrition was determined in sterile grown wild type Arabidopsis thaliana plants (wt) and plants lacking the expression of the lysine-histidine transporter 1 (T27). Plants were grown on ½ strength MS media in which all N was exchanged for 3 mM NO3- and either 30 *µ*M of either L-alanine or L-glutamine. The supplied amino acids were labelled (98%) with 15N so that uptake of N from these sources could be distinguished from uptake of nitrate. The results are shown in Figure 3. L-alanine N and L-glutamine were observed to make a reduced contribution to growth in plants which lacked expression of the lysine-histidine transporter 1.

Wild type and transgenic A. *thaliana* plants carrying the E.coli gene cycA were grown in sterile agar media based on ½ strength MS media in which nitrogen was supplied as Glycine. Glycine mediated growth of the wild type and transgenic plants was compared (Fig 4).

The transgenic plants were observed to show increased glycine mediated growth, relative to wild type controls.

**Table 1**

| **Enzyme** | **Accession number (Brenda protein database)** | **Source organism** | **Substrate** |
|---|---|---|---|
| ***Dehydratase*** | | | |
| D-Serine dehydratase | EC-number 4.2.1.14 | -E. intermedia | -D-Ser |
| | | -E. coli | -D-Thr |
| | | -Klebsiella pneumoniae | -D-allothreonine |
| | | -Chicken | |

| ***Oxidases*** | | | |
|---|---|---|---|
| D-Amino acid | EC-number | -Pig | -Most D-amino acid |
| oxidase | 1.4.3.3 | -Human | |
| | | -Rat | |
| | | -Candida tropicalis | |
| | | -Trigonopsis variabilis | |
| | | -Neuspora crassa | |
| | | -Chlorella vulgaris | |
| | | -Rhodotorula gracilis | |
| D-Glutamate oxidase | EC-number 1.4.3.7 | -Octopus vulgaris | -D-Asp |
| | | | -D-Glu |
| | | -Orconectes limosus | |
| -D-Aspartate oxidase | EC-number 1.4.3.1 | -Rabbit | -D-Asp |
| | | -Human | -D-Glu |
| | | -Pig | -N-methyl-D-Asp |
| | | -Bovine | -Meso-2,3-diaminosuccenate (relevancy not known) |
| | | -Bos taurus | |
| | | | -cis-Thiazolidine-2,4- |
| | | | dicarboxylate (relevancy not known) |

| ***Racemases*** | | | |
|---|---|---|---|
| D-Glutamate racemase | EC-number 5.1.1.3 | -Lactobacillus sp | -D/L-Glu |
| | | -Pediococcus pentosaceus | |
| | | -E. coli | |

| Transaminases | | | |
|---|---|---|---|
| D-Methionine transaminase | EC-number 2.6.1.21 | -Brassica sp mitochondria | -D-Methionine |
| D-Alanine transaminase | EC-number 2.6.1.21 | -Bacillus sp. | -D-Arg |
| | | -Listeria monocytogenes | -D-Ala |
| | | | -D-Asp |
| | | -Thermophilic bacterium | -D-Glu |
| | | | -D-Lue |
| | | | -D-Lys |
| | | | -D-Met |
| | | | -D-Phe |
| | | | -D-Norvaline |
| | | | - |

**Table 2**

| Gene | Species | Genbank Entry | Reference No | Reference No |
|---|---|---|---|---|
| Aux-1 | *Arabidopsis thaliana* | CAA67308.1 | Q96247 | 2.A.18.1.1 |
| | | GI:1531758 | | |
| AAP1 | *Arabidopsis thaliana* | CAA47603.1 | Q42400 | 2.A.18.2.1 |
| | | GI:22641 | | |
| LHT1 | *Arabidopsis thaliana* | AAC49885.1 | 024405 | 2.A.18.2.2 |
| | | GI:2576361 | | |
| AAP3 | *Arabidopsis thaliana* | CAA54630.1 | Q39134 | 2.A.18.2.3 |
| | | GI:3970652 | | |
| AAP6 | *Arabi dopsi s thaliana* | CAA65051.1 | P92934 | 2.A.18.2.4 |
| | | GI:1769887 | | |
| AAP8 | *Arabidopsis thaliana* | | 080592 | 2.A.18.2.5 |
| Prt1 | *Arabidopsis thaliana* | CAA65052.1 | P92961 | 2.A.18.3.1 |
| | | GI:1769901 | | |
| ProT1 | Lycopersicon *esculentum* | | Q9XE48 | 2.A.18.3.2 |
| MTR aka AAP1 | Neurospora crassa | P38680 | P38680 | 2.A.18.4.1 |
| | | GI:2507070 | | |
| GAP1 | *Mycoplasma pneumoniae* | NP_110007.1 | GeneID: 876937 | |
| | | GI:13508058 | | |
| Bap2p | *Saccharomyces cerevisiae* | NP_009624.1 | GeneID: 852360 | |
| | | GI:6319542 | | |
| cycA | *Escherichia coli* | NP_418629.1 | GeneID: 948725 | |
| | | GI:16132030 | | |

## Claims

1. A method of increasing the growth of a plant comprising:
increasing in the plant the expression of a nucleic acid encoding an amino acid transporter that mediates the uptake of amino acids from the environment into said plant,and;
exposing the plant to a growth medium comprising one or more amino acids whose uptake is mediated by the amino acid transporter,
the growth of said plant being increased following said expression.

2. A method according to claim 1 wherein the nucleic acid is operably linked to a heterologous regulatory element.

3. A method according to claim 1 or claim 2 wherein the nucleic acid is a heterologous nucleic acid.

4. A method according to any one of the preceding claims wherein the amino acid transporter is specifically expressed in the root tissue of the plant.

5. A method according to claim 4 wherein said amino acid transporter is specifically expressed in the root hair of the plant.

6. A method according to any one of the preceding claims comprising exposing the roots of the plant to the growth medium.

7. A method according to any one of the preceding claims comprising adding to the growth medium one or more amino acids whose uptake is mediated by the amino acid transporter.

8. A method according to any one of the preceding claims wherein the one or more amino acids are the sole nitrogen source in the growth medium.

9. A method according to any one of the preceding claims wherein the amino acid transporter is a plant amino acid transporter.

10. A method according to claim 9 wherein the amino acid transporter is an AAP amino acid transporter.

11. A method according to claim 10 wherein the amino acid transporter is an AAP1, AAP3, AAP6 or AAP8 amino acid transporter.

12. A method according to claim 9 wherein the amino acid transporter is an LHT amino acid transporter.

13. A method according to claim 12 wherein the amino acid transporter is an LHT1 amino acid transporter.

14. A method according to claim 13 wherein the LHT1 amino acid transporter is the Arabidopsis LHT1.

15. A method according to any one of claims 1 to 8 wherein the amino acid transporter is a bacterial amino acid transporter.

## Patentansprüche

1. Verfahren zur Steigerung des Wachstums einer Pflanze, das Folgendes umfasst:
die Steigerung der Expression einer Nucleinsäure in der Pflanze, die für einen Aminosäurentransporter kodiert, der die Aufnahme von Aminosäuren aus der Umgebung in die Pflanze vermittelt, und
das Aussetzen der Pflanze gegenüber einem Wachstumsmedium, das eine oder mehrere Aminosäuren umfasst, deren Aufnahme durch den Aminosäuretransporter vermittelt wird,
wobei Wachstum der Pflanze nach der Expression erhöht ist.

2. Verfahren nach Anspruch 1, worin die Nucleinsäure operabel an ein heterologes Regulationselement gebunden ist.

3. Verfahren nach Anspruch 1 oder 2, worin die Nucleinsäure eine heterologe Nucleinsäure ist.

4. Verfahren nach einem der vorangegangenen Ansprüche, worin der Aminosäuretransporter spezifisch im Wurzelgewebe der Pflanze exprimiert wird.

5. Verfahren nach Anspruch 4, worin der Aminosäuretransporter spezifisch im Wurzelhaar der Pflanze exprimiert wird.

6. Verfahren nach einem der vorangegangenen Ansprüche, umfassend das Aussetzen der Wurzeln der Pflanze gegenüber einem Wachstumsmedium.

7. Verfahren nach einem der vorangegangenen Ansprüche, das das Zusetzen einer oder mehrerer Aminosäuren, deren Aufnahme durch den Aminosäuretransporter vermittelt wird, zu dem Wachstumsmedium umfasst.

8. Verfahren nach einem der vorangegangenen Ansprüche, worin die eine Aminosäure oder die mehreren Aminosäuren die einzige(n) Stickstoffquelle(n) in dem Wachstumsmedium ist/sind.

9. Verfahren nach einem der vorangegangenen Ansprüche, worin der Aminosäuretransporter ein pflanzlicher Aminosäuretransporter ist.

10. Verfahren nach Anspruch 9, worin der Aminosäuretransporter ein AAP-Aminosäuretransporter ist.

11. Verfahren nach Anspruch 10, worin der Aminosäuretransporter ein AAP1-, AAP3-, AAP6- oder AAP8-Aminosäuretransporter ist.

12. Verfahren nach Anspruch 9, worin der Aminosäuretransporter ein LHT-Aminosäuretransporter ist.

13. Verfahren nach Anspruch 12, worin der Aminosäuretransporter ein LHT1-Aminosäuretransporter ist.

14. Verfahren nach Anspruch 13, worin der LHT1-Aminosäuretransporter Arabidopsis LHT1 ist.

15. Verfahren nach einem der Ansprüche 1 bis 8, worin der Aminosäuretransporter ein bakterieller Aminosäuretransporter ist.

## Revendications

1. Procédé pour augmenter la croissance d'une plante, qui consiste à :
augmenter dans la plante l'expression d'un acide nucléique codant pour un transporteur d'acides aminés qui médie le captage des acides aminés de l'environnement dans ladite plante, et ;
exposer la plante à un milieu de croissance comprenant un ou plusieurs acides aminés dont le captage est médié par le transporteur d'acides aminés,
la croissance de ladite plante étant augmentée suite à ladite expression.

2. Procédé selon la revendication 1, dans lequel l'acide nucléique est en liaison fonctionnelle avec un élément régulateur hétérologue.

3. Procédé selon la revendication 1 ou la revendication 2, dans lequel l'acide nucléique est un acide nucléique hétérologue.

4. Procédé selon l'une quelconque des revendications précédentes, dans lequel le transporteur d'acides aminés est spécifiquement exprimé dans le tissu racinaire de la plante.

5. Procédé selon la revendication 4, dans lequel ledit transporteur d'acides aminés est spécifiquement exprimé dans les poils absorbants de la plante.

6. Procédé selon l'une quelconque des revendications précédentes, qui consiste à exposer les racines de la plante au milieu de croissance.

7. Procédé selon l'une quelconque des revendications précédentes, qui consiste à ajouter au milieu de croissance un ou plusieurs acides aminés dont le captage est médié par le transporteur d'acides aminés.

8. Procédé selon l'une quelconque des revendications précédentes, dans lequel le ou plusieurs acides aminés sont la seule source d'azote dans le milieu de croissance.

9. Procédé selon l'une quelconque des revendications précédentes, dans lequel le transporteur d'acides aminés est un transporteur d'acides aminés de plante.

10. Procédé selon la revendication 9, dans lequel le transporteur d'acides aminés est un transporteur d'acides aminés AAP.

11. Procédé selon la revendication 10, dans lequel le transporteur d'acides aminés est un transporteur d'acides aminés AAP1, AAP3, AAP6 ou AAP8.

12. Procédé selon la revendication 9, dans lequel le transporteur d'acides aminés est un transporteur d'acides aminés LHT.

13. Procédé selon la revendication 12, dans lequel le transporteur d'acides aminés est un transporteur d'acides aminés LHT1.

14. Procédé selon la revendication 13, dans lequel le transporteur d'acides aminés LHT1 est le LHT1 d'Arabidopsis.

15. Procédé selon l'une quelconque des revendications 1 à 8, dans lequel le transporteur d'acides aminés est un transporteur d'acides aminés bactérien.
